# FASCICULE DE BREVET EUROPEEN

(11) **EP 0 693 951 B1**
(45) Date de publication et mention de la délivrance du brevet: **26.08.1998**
(21) Numéro de dépôt: 94911217.1
(22) Date de dépôt: 24.03.1994
(51) Int. Cl.: A61N 1/30

(54) **APPLICATEUR D'IMPULSIONS ELECTRIQUES POUR TRAITEMENT DE TISSUS BIOLOGIQUES**
EINRICHTUNG ZUR ABGABE VON ELEKTRISCHEN IMPULSEN FÜR DIE BEHANDLUNG BIOLOGISCHER GEWEBE
DEVICE FOR THE APPLICATION OF ELECTRICAL PULSES IN THE TREATMENT OF BIOLOGICAL ISSUES

(30) Priorité: 30.03.1993 FR 9303688
(43) Date de publication de la demande: 31.01.1996
(73) Titulaire: CENTRE NATIONAL DE LA RECHERCHE SCIENTIFIQUE (CNRS), 75794 Paris Cédex 16 (FR)
(72) Inventeur: MIR, Lluis, M., F-91370 Verrières-le-Buisson (FR); ORLOWSKI, Stéphane, F-92160 Antony (FR); SIROS, Michel, F-92160 Antony (FR)
(74) Mandataire: Michelet, Alain
(86) Numéro de dépôt international: FR9400330
(87) Numéro de publication internationale: WO9422526

(56) Documents cités:
- EP-A- 0 378 132
- DE-C- 863 111
- GB-A- 1 653 819
- COMPTE RENDU ACADEMIE DES SCIENCES, vol.313, no.111, 27 Novembre 1991, PARIS pages 613 - 618 MIR ET AUTRES 'l'électrochimiotherapie,un nouveau traitement antitumoral : premier essai clinique' cité dans la demande

## Description

La présente invention concerne un dispositif permettant l'application d'impulsions électriques pour le traitement de tissus biologiques. Elle est plus particulièrement destinée à la mise en oeuvre de l'électrochimiothérapie.

L'électrochimiothérapie est une nouvelle méthode thérapeutique qui a plus particulièrement été développée pour le traitement des cancers.

En effet, on a constaté qu'un certain nombre de substances actives traversent difficilement la membrane des cellules auxquelles elles sont destinées.

Pour surmonter cette difficulté, il est souvent nécessaire d'augmenter les doses de substances apportées, ce qui est souvent difficile et présente presque toujours des effets secondaires néfastes.

Depuis peu, on a montré que l'application d'impulsions électriques à des cellules permet d'augmenter la perméabilité de leur membrane plasmique.

L'électrochimiothérapie exploite cette possibilité en combinant l'apport d'une substance active avec l'application d'impulsions électriques brèves et intenses. La pénétration de la substance active dans les cellules soumises au champ électrique est alors facilitée. On a, par exemple, utilisé l'électrochimiothérapie pour le traitement de tumeurs par la bléomycine. Les effets antitumoraux de la bléomycine combinée aux impulsions électriques peuvent être potentialisés par des injections d'immunostimulants comme les interleukines, par exemple l'interleukine-2, ou par des injections de compositions médicamenteuses comme des cellules syngéniques, allogéniques ou xénogéniques sécrétrices d'interleukines, par exemple l'interleukine-2. L'injection de ces substances ou compositions est surtout efficace, si elle est faite localement au niveau de la tumeur, préalablement traitée par l'électrochimiothérapie.

Jusqu'à présent, les champs électriques nécessaires à la mise en oeuvre de l'électrochimiothérapie étaient obtenus par l'application d'impulsions entre deux électrodes externes placés, dans toute la mesure du possible, de part et d'autre de la tumeur à traiter.

Le contact électrique de ces électrodes avec la peau est assuré par un gel conducteur.

Une telle technique est décrite par exemple dans le Compte-Rendu de l'Académie des Sciences de Paris t.313, série III, pages 613-618, 1991 : "L'électrochimiothérapie, un nouveau traitement anti-tumoral : premier essai clinique" Lluis M. MIR et al.

Les électrodes utilisées jusqu'à présent pour l'application des impulsions électriques ne permettent pas d'assurer une répartition homogène des champs électriques produits sur l'ensemble du volume à traiter et nécessitent, pour la partie chimiothérapeutique, l'injection du produit médicamenteux antitumoral dans tout le corps du patient.

Le but de la présente invention est un applicateur permettant d'améliorer la répartition et le contrôle des champs électriques produits pour l'électrochimiothérapie.

C'est un autre objet de l'invention de proposer un applicateur d'impulsions électriques qui puisse être utilisé en évitant ou en limitant les effets secondaires produits.

C'est encore un but de l'invention de proposer un applicateur d'impulsions électriques pour électrochimiothérapie qui puisse être utilisé à plusieurs reprises sans causer de lésions gênantes aux tissus sains proches de la tumeur, par exemple la peau.

C'est aussi un but de l'invention de proposer un dispositif de faibles dimensions, stérilisable, autoclavable et pouvant supporter des courants intenses.

A cet effet, l'invention concerne un applicateur d'impulsions électriques pour le traitement de tissus biologiques permettant l'application d'un champ électrique aux cellules des tissus biologiques, de manière à modifier les propriétés de leur membrane. Cet applicateur comprend des électrodes et un générateur d'impulsions.

Selon l'invention, les électrodes comportent au moins trois aiguilles destinées à être introduites dans les tissus à traiter et à définir un volume de traitement, lesdites aiguilles formant deux à deux des couples d'aiguilles, et un commutateur d'aiguilles adressant les impulsions produites par le générateur d'impulsions, successivement sur les différents couples d'aiguilles.

Dans différents modes de réalisation préférés, l'applicateur d'impulsions électriques pour le traitement de tissus biologiques de l'invention comporte les caractéristiques suivantes, selon toutes leurs combinaisons techniquement possibles :
- les aiguilles des électrodes sont fixées, de manière interchangeable, sur un applicateur d'aiguilles ;
- les aiguilles comportent chacune une embase et une tige terminée par une pointe, l'embase assurant la fixation de l'aiguille sur le porte-aiguilles, la pointe assurant la pénétration de l'aiguille dans les tissus, une ou plusieurs parties de la tige étant entourées d'une gaine isolante ;
- une partie des gaines isolantes des aiguilles est destinée à rester implantée dans les tissus ;
- les électrodes comportent une aiguille centrale et six aiguilles périphériques, régulièrement réparties sur un cercle centré sur l'aiguille centrale ;
- la tension des impulsions appliquées est proportionnelle à la distance séparant les deux aiguilles entre lesquelles elle est appliquée ;
- chaque aiguille est séparée de ses voisines d'une distance comprise entre 4 et 10 mm, et de préférence égale à 6,5 mm ;
- chaque impulsion électrique est une impulsion rectangulaire, d'amplitude comprise entre 100 et 1500 V et de largeur temporelle comprise entre 10 et 200 µs ;
- les aiguilles des électrodes sont creuses et permettent l'injection de la substance injectée, localement dans le volume de traitement ;
- le porte-aiguilles porte une seringue formée d'un corps et d'un piston associée à chaque aiguille ;
- le commutateur comprend deux relais associés à chaque aiguille permettant de la connecter éventuellement, soit à la borne positive, soit à la borne négative du générateur d'impulsions ;
- l'applicateur comporte un pilote permettant, après une injection de substance, la réalisation d'une séquence d'impulsions électriques quelconque déterminée par l'opérateur.

Un mode de réalisation de l'invention sera décrit plus en détail en référence aux dessins dans lesquels :

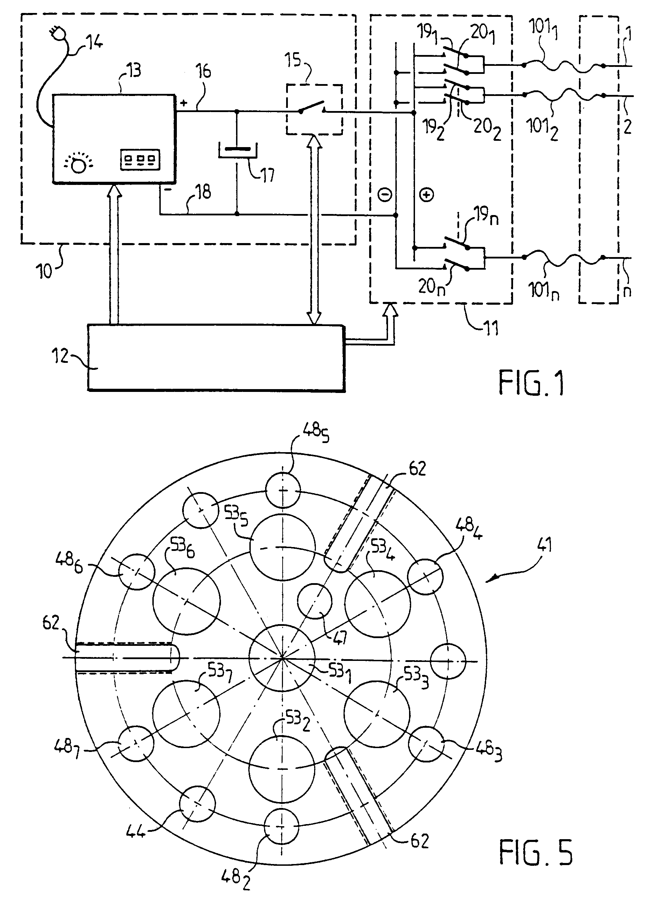

- la Figure 1 est une représentation schématique du circuit électrique général de l'invention ;

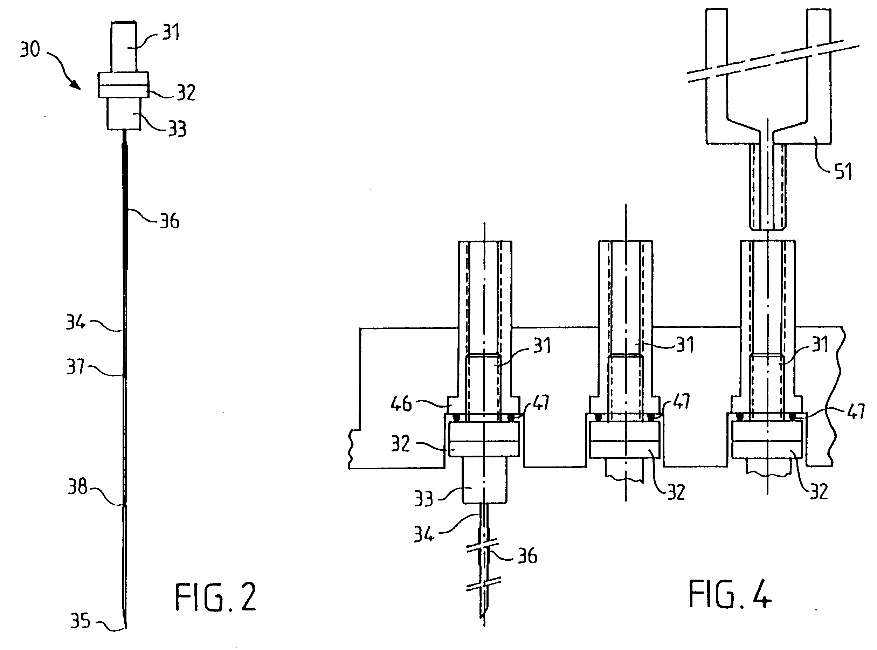

- la Figure 2 représente une vue en coupe d'une aiguille ;

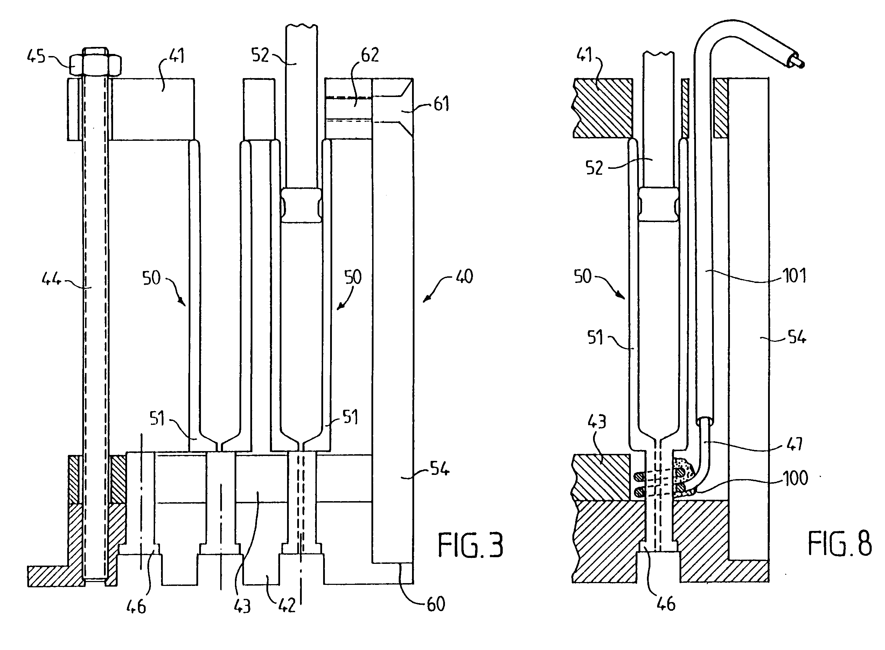

- la Figure 3 représente une vue partielle en coupe d'un porte-aiguilles ;
- la Figure 4 représente une vue en coupe de la fixation d'une aiguille sur le porte-aiguilles ;
- la Figure 5 est une vue du dessus de la plaquette supérieure du porte-aiguilles ;
- la Figure 6 est une vue du dessus du fond du porte-aiguilles ;
- la Figure 7 est une vue du dessus de la rondelle isolante du porte-aiguilles ;

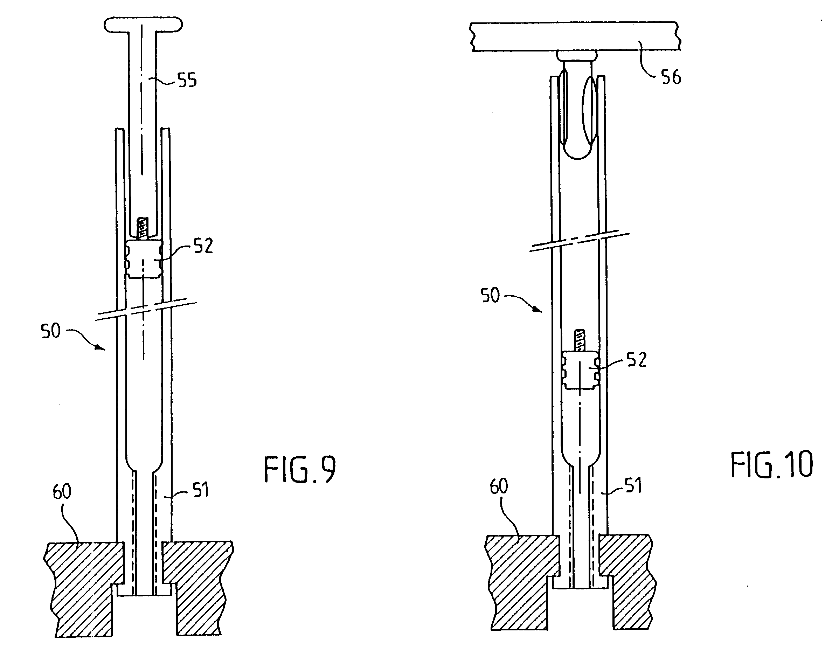

- la Figure 8 est une vue en coupe du porte-aiguilles faisant apparaître la liaison électrique de l'aiguille avec le générateur d'impulsions ;
- la Figure 9 est une vue en coupe partielle d'une seringue à corps conducteur en état d'injection ;
- la Figure 10 est une vue en coupe partielle d'une seringue à corps conducteur en état d'application du champ électrique.

Toutes les aiguilles ayant la même structure et étant associées aux mêmes éléments, on comprend que sur les Figures, une seule référence numérique a été utilisée pour désigner chacun de ces éléments quelle que soit l'aiguille auquel il est destiné. Cette référence a éventuellement été précisée avec un indice correspondant à la référence de l'aiguille.

L'applicateur d'impulsions électriques pour électrochimiothérapie de tissus biologiques est destiné à permettre l'application d'un champ électrique variable à des cellules situées entre une paire d'aiguilles 1, 2... n.

A cet effet, il comporte un générateur d'impulsions 10, un commutateur 11 et un pilote 12. Le générateur d'impulsions 10 comporte une alimentation haute tension 13 reliée au secteur par le cordon 14, et au commutateur par l'intermédiaire d'un interrupteur 15 placé sur sa sortie positive 16, et d'un condensateur 17 placé en parallèle entre sa sortie positive 16 et sa sortie négative 18.

Chaque électrode 1, 2... n est susceptible d'être reliée soit au pôle positif 16 de l 'alimentation haute tension 13, soit à son pôle négatif 18 par l'intermédiaire de deux relais 19₁, 20₁, 19₂, 20₂,... 19ₙ, 20ₙ appartenant au commutateur 11.

Le pilote 12 commande en fonction des instructions qui lui sont fournies par un opérateur ou par l'intermédiaire d'un programme, l'alimentation haute tension 13, l'interrupteur 15 et le commutateur 11.

Ainsi, l'applicateur d'impulsions électriques permet d'appliquer entre les aiguilles 1, 2... n, prises deux à deux selon toutes leurs combinaisons possibles, des cycles d'impulsions préalablement déterminés.

Ces cycles peuvent être déterminés par tous moyens, en particulier expérimentaux, de manière à fournir les meilleurs résultats possibles.

Les relais 19₁, 20₁, ... 19ₙ, 20ₙ sont de préférence chacun formés par un relais à verge ou ampoule REED, dont l'excitation est produite soit par le déplacement mécanique d'un petit aimant dont la position est asservie, soit par une commande classique au moyen d'une bobine. Ce déplacement est produit par un système classique d'asservissement de position réalisé par une bobine.

Cette disposition permet d'obtenir un encombrement minimum du commutateur 11.

A titre d'exemple, la fermeture du relais 19₁ et du relais 20ₙ permet, lorsque l'interrupteur 15 est fermé, d'adresser une impulsion entre les électrodes 1 et n, l'électrode 1 étant l'électrode positive et l'électrode n, l'électrode négative.

Le contact électrique étant établi par les électrodes, sur toute leur longueur non isolée, avec les tissus, le champ ainsi produit s'étend en profondeur dans ces tissus. Il est donc possible de soumettre à des champs électriques des cellules qui n'auraient pas été accessibles, ou pas aisément accessibles avec des électrodes simplement posées à la surface des tissus.

Les impulsions appliquées à chaque couple d'aiguilles sont de préférence des impulsions, rectangulaires, d'amplitude comprise entre 100 et 1500 V, et de largeur temporelle comprise entre 10 et 200 µs. Ces impulsions sont espacées, pour chaque couple d'aiguilles, d'une durée réglable comprise entre 0,2 et 2 s, et de préférence de 1 s.

Pour chaque couple d'aiguilles, on pourra par exemple appliquer huit impulsions successives de même polarité, ou encore quatre impulsions d'une première polarité suivies ultérieurement dans le cycle de quatre impulsions de polarité inversée. Dans le cas d'électrodes comportant de nombreuses aiguilles, par exemple sept comme dans l'exemple de réalisation que nous décrirons plus loin, les séquences d'impulsions concernant les différents couples d'aiguilles peuvent être imbriquées. Ainsi, compte tenu de la durée de chaque impulsion et de celle qui doit séparer deux impulsions successives appliquées à un même couple d'électrodes, il est possible d'exciter les différents couples d'électrodes les uns après les autres tout en respectant ces séquences.

Les champs électriques ainsi produits peuvent être approximativement uniformément répartis, y compris en profondeur, puisque les aiguilles pénètrent dans les tissus et définissent donc un ensemble de volumes élémentaires de tissus, chacun de ces volumes étant compris entre deux électrodes d'un couple. L'application successive de champs électriques à ces volumes élémentaires permet d'obtenir une bonne uniformité de traitement sur l'ensemble du volume de tissus biologiques traités.

Chaque aiguille 1, 2... n comporte une embase 30, une tête 31, une prise 32 comprenant un méplat et une base 33.

La base 33 porte la tige 34 qui se termine par une pointe 35. Une ou plusieurs parties de la tige 34 comportent de préférence un manchon 36 isolant, par exemple en PTFE (polytétrafluorure d'éthylène), qui permet, lorsqu'elle est insérée dans les tissus, d'éviter l'application des impulsions électriques à certaines zones. En particulier, il est souvent préférable d'éviter l'application du champ électrique en surface.

D'autre part, selon un mode de réalisation préféré, la partie de cette gaine isolante 36, au voisinage de l'embase, est amovible et est susceptible de rester un certain temps dans les tissus pour faciliter l'utilisation de l'applicateur d'impulsions à plusieurs reprises pour le traitement du même volume de tissu, sans risquer d'endommager les tissus sains superficiels et, le traitement électrique étant terminé, au moins provisoirement, pour continuer l'injection d'une ou de plusieurs substances, ou compositions médicamenteuses (par exemple des immunomodulateurs, tels que l'interleukine-2 ou des cellules sécrétrices par exemple d'interleukine-2). L'intérêt du cathéter ainsi formé est aussi que sa souplesse permet un port prolongé sans lésions mécaniques des tissus.

Les aiguilles sont avantageusement fixées sur un porte-aiguilles 40. Ce porte-aiguilles ayant, par exemple, une section de forme générale circulaire, comprend une plaquette supérieure 41, une plaquette de fond 42 et une rondelle isolante 43. La plaquette isolante 43 repose sur la plaquette de fond 42 qui est reliée à la plaquette supérieure par des colonnettes 44 formant entretoises. Ces colonnettes 44 sont vissées dans la plaquette de fond 42 et reçoivent des boulons 45 assurant la fixation de la plaquette supérieure 41. Des inserts 46 sont placés dans des ouvertures prévues à cet effet dans la plaquette de fond et la plaquette isolante, et ils sont destinés à recevoir les têtes 31 des aiguilles. Les têtes 31 peuvent par exemple être filetées, les inserts 46 portant des filetages complémentaires. Un outil externe, non représenté, est susceptible de coopérer avec les méplats de la zone 32 des aiguilles, et peut par exemple être utilisé pour faciliter le montage et le démontage des aiguilles dans les inserts 46.

Une liaison électrique 100 établit la liaison de l'insert 46 par l'intermédiaire d'un fil 101 avec le commutateur 11. Un joint 47 assure avantageusement une bonne liaison entre des aiguilles 1, 2... n et les inserts 46.

Par l'intermédiaire de l'insert 46 sur lequel elle est vissée, chaque aiguille 1, 2... n est donc ainsi reliée au commutateur 11 et donc susceptible d'être reliée au générateur d'impulsions 10.

La plaquette supérieure comporte des passages 44 pour les colonnettes et des passages 48 pour les fils électriques.

La rondelle isolante comporte des passages 44 pour les colonnettes et des passages 49 permettant d'assurer sa fixation sur la plaquette de fond.

Dans le mode de réalisation préféré, des aiguilles 1, 2... n sont creuses et permettent d'assurer l'injection locale d'une substance active localement.

A cet effet, les aiguilles 1, 2... n sont creuses et reliées par l'intermédiaire des inserts 46 à des seringues 50 comportant un corps 51 et un piston 52. La plaquette supérieure 41 en appui sur les corps 51 des seringues contribue à les maintenir en position.

Cette plaquette 41 comporte des passages 53 permettant la libre circulation des pistons 52 des seringues 50.

Un cylindre extérieur 54 est avantageusement prévu et fixé d'une part sur la plaquette supérieure 41, et d'autre part sur la plaquette de fond 42. Ce cylindre extérieur entoure le dispositif, assure sa protection et facilite sa manipulation. Ce cylindre extérieur 54 est d'une part en appui sur la plaquette de fond 42 qui comporte un épaulement 60 prévu à cet effet, et d'autre part vissé sur la plaquette supérieure 41 par une vis 61 et coopérant avec un filetage 62 aménagé dans la plaquette supérieure 41.

La tige creuse 34 de l'aiguille comporte au moins une ouverture à son extrémité 35. Afin d'améliorer la répartition homogène de la substance injectée dans tout le volume de tissu traité, elle comporte de préférence également des ouvertures 37, 38 réparties à des niveaux intermédiaires sur sa hauteur. Une telle aiguille est qualifiée de fenestrée.

Dans un autre mode de réalisation, les corps de seringues 51 sont conducteurs, par exemple métalliques, ce qui permet de simplifier les liaisons électriques.

Les pistons 52 sont actionnés par des tiges 55 amovibles. Elles sont par exemple vissées sur le piston 52 et dévissables.

L'alimentation électrique des aiguilles est alors faite par l'intermédiaire des corps de seringues 51. Leur partie supérieure forme un contact électrique femelle qui coopère avec un porte-prise mâle 56 relié au commutateur 11.

Les tiges 55 sont d'abord fixées sur les pistons 52 pour permettre l'injection. Elles sont ensuite démontées et le porte-prise est connecté sur les corps de seringues 51. L'application des impulsions électriques peut alors être réalisée.

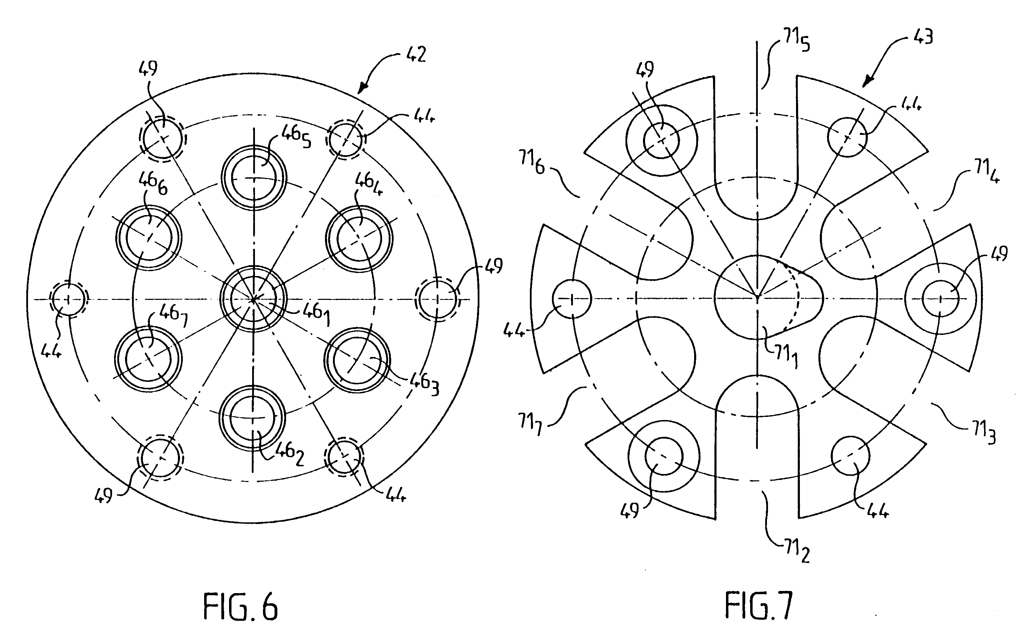

Les Figures 5, 6 et 7 font apparaître la répartition des aiguilles et des seringues lors de l'utilisation simultanée de sept d'entre elles. On dispose alors l'une d'entre elles 1 au centre et les six autres 2-7 sont également réparties sur un cercle centré sur la première.

On pourra utiliser avantageusement des répartitions dérivées lors de la mise en oeuvre d'un plus grand nombre d'aiguilles, par exemple l'utilisation de dix-neuf aiguilles pourra être réalisée en plaçant les douze aiguilles complémentaires sur une deuxième couronne concentrique du premier cercle, ayant un rayon approximativement double de celui-ci, et encore une répartition de trente-sept aiguilles peut être obtenue en répartissant les dix-huit aiguilles additionnelles par rapport à la configuration précédente, uniformément sur une troisième couronne concentrique du premier cercle et ayant un rayon environ trois fois supérieur au premier cercle, de façon qu'il y ait toujours équidistance dans tous les couples constitués par une aiguille quelconque et toutes celles qui l'entourent.

Lorsque les aiguilles ne sont pas équidistantes, la tension de l'impulsion appliquée sera fonction de l'écartement du couple d'aiguilles auquel elle est destinée. Cette tension est de préférence proportionnelle à l'écartement.

De manière générale, les caractéristiques des impulsions électriques peuvent être déterminées en vue d'un protocole particulier. Elles ne sont pas nécessairement rectangulaires.

L'applicateur d'impulsions électriques peut donc être utilisé de la manière suivante. Les seringues 50 sont tout d'abord remplies de la solution comprenant la substance active à injecter.

L'appareil est alors mis en place par introduction des aiguilles dans les tissus à traiter.

La substance active est injectée.

On attend alors pendant une durée fixée par l'expérience, puis par déclenchement du pilote 12, les séquences d'impulsion électriques sont activées et produisent les intensités voulues de champ électrique au sein des tissus placés entre les aiguilles ou à leur voisinage. A la fin du traitement électrique, les aiguilles sont retirées et les gaines isolantes peuvent être éventuellement laissées en place pour servir ultérieurement à injecter des substances ou des compositions médicamenteuses.

Par rapport au mode de réalisation décrit plus haut, différentes variations sont envisageables sans sortir du cadre de l'invention. En particulier, la commande du générateur peut être réalisée à distance et, par exemple, à partir d'un déclencheur placé sur l'applicateur.

Par ailleurs, la forme des impulsions électriques pourrait être modifiée et adaptée en fonction de résultats expérimentaux.

Les signes de référence insérés après les caractéristiques techniques mentionnées dans les revendications, ont pour seul but de faciliter la compréhension de ces dernières, et n'en limitent aucunement la portée.

## Revendications

1. Applicateur d'impulsions électriques pour le traitement de tissus biologiques permettant l'application d'un champ électrique aux cellules des tissus biologiques de manière à modifier les propriétés de leur membrane, comprenant des électrodes et un générateur d'impulsions (10),
caractérisé en ce que les électrodes comportent au moins trois aiguilles (1, 2... n) destinées à être introduites dans les tissus à traiter et à définir un volume de traitement, lesdites aiguilles (1, 2... n) formant deux à deux des couples d'aiguilles, et un commutateur (11) d'aiguilles adressant les impulsions produites par le générateur d'impulsions (10), successivement sur les différents couples d'aiguilles.

2. Applicateur d'impulsions électriques selon la revendication 1, caractérisé en ce que les aiguilles (1, 2... n) des électrodes sont fixées de manière interchangeable sur un applicateur d'aiguilles (40).

3. Applicateur d'impulsions électriques selon la revendication 2, caractérisé en ce que les aiguilles (1, 2... n) comportent chacune une embase (30) et une tige (34) terminée par une pointe, l'embase (30) assurant la fixation de l'aiguille sur le porte-aiguilles (40), la pointe (35) assurant la pénétration de l'aiguille dans les tissus, une ou plusieurs parties de la tige étant entourées d'une gaine isolante (36).

4. Applicateur d'impulsions électriques selon la revendication 3, caractérisé en ce qu'une partie des gaines isolantes (36) des aiguilles (1, 2... n) est destinée à rester implantée dans les tissus.

5. Applicateur d'impulsions électriques selon l'une quelconque des revendications 1 à 4, caractérisé en ce que les électrodes comportent une aiguille centrale (1) et six aiguilles périphériques (2-n), régulièrement réparties sur un cercle centré sur l'aiguille centrale (1).

6. Applicateur d'impulsions électriques selon l'une quelconque des revendications 1 à 5, caractérisé en ce que la tension des impulsions appliquées est proportionnelle à la distance séparant les deux aiguilles entre lesquelles elle est appliquée.

7. Applicateur d'impulsions électriques selon l'une quelconque des revendications 1 à 6, caractérisé en ce que chaque aiguille est séparée de ses voisines d'une distance comprise entre 4 et 10 mm, et de préférence égale à 6,5 mm.

8. Applicateur d'impulsions électriques selon l'une quelconque des revendications 1 à 7, caractérisé en ce que chaque impulsion électrique est une impulsion rectangulaire, d'amplitude comprise entre 100 et 1500 V et de largeur temporelle comprise entre 10 et 200 µs.

9. Applicateur d'impulsions électriques selon l'une quelconque des revendications 1 à 8, caractérisé en ce que les aiguilles (1, 2... n) des électrodes sont creuses et permettent l'injection de la substance active, localement dans le volume de traitement.

10. Applicateur d'impulsions électriques selon la revendication 9, caractérisé en ce que les électrodes sont fenestrées.

11. Applicateur d'impulsions électriques selon la revendication 10, caractérisé en ce que le porte-aiguilles (40) porte une seringue (50) formée d'un corps (51) et d'un piston (52) associée à chaque aiguille.

12. Applicateur d'impulsions électriques selon l'une quelconque des revendications 1 à 11, caractérisé en ce que le commutateur (11) comprend deux relais (19₁, 20₁...) associés à chaque aiguille (1) permettant de la connecter éventuellement, soit à la borne positive, soit à la borne négative du générateur d'impulsions (10).

13. Applicateur d'impulsions électriques selon l'une quelconque des revendications 8 à 12, caractérisé en ce qu'il comporte un pilote (12) permettant, après une injection de substance, la réalisation d'une séquence d'impulsions électriques quelconque déterminée par l'opérateur.

## Claims

1. Electric pulse applicator for the treatment of biological tissue allowing an electric field to be applied to cells of biological tissue in such a way as to modify the properties of their membrane, comprising electrodes and a pulse generator (10),
characterized in that the electrodes comprise at least three needles (1, 2, ..., n) intended to be introduced into the tissue to be treated and which define a treatment volume, said needles (1, 2, ..., n) in twos forming pairs of needles, and a needle selector switch (11) which sends the pulses produced by the pulse generator (10) successively onto the different pairs of needles.

2. Electric pulse applicator according to claim 1, characterized in that the needes (1, 2, ..., n) of the electrodes are fixed onto a needle applicator (40) in an interchangeable way.

3. Electric pulse applicator according to claim 2, characterized in that the needles (1, 2, ..., n) each comprise a base (30) and a stem (34) terminated by a point, the base (30) ensuring the fixing of the needle onto the needle holder (40), the point (35) ensuring the penetration of the needle into the tissue, one or more portions of the stem being surrounded by an insulating sleeve (36).

4. Electric pulse applicator according to claim 3, characterized in that a part of the insulating sleeves (36) of the needles (1, 2 ..., n) is intended to remain implanted in the tissue.

5. Electric pulse applicator according to any one of claims 1 to 4, characterized in that the electrodes comprise a central needle (1) and six peripheral needles (2-n) regularly distributed on a circle centered on the central needle (1).

6. Electric pulse applicator according to any one of claims 1 to 5, characterized in that the voltage of the applied pulses is proportional to the distance separating the two needles between which it is applied.

7. Electric pulse applicator according to any one of claims 1 to 6 characterized in that each needle is separated from its neighbours by a distance of 4 to 10 mm, and preferably 6.5 mm.

8. Electric pulse applicator according to any one of claims 1 to 7, characterized in that each electric pulse is a rectangular pulse of amplitude 100 to 1500 V and pulse length 10 to 200 µs.

9. Electric pulse applicator according to any one of claims 1 to 8, characterized in that the needles (1, 2, ..., n) of the electrodes are hollow and allow the active substance to be injected locally into the treatment volume.

10. Electric pulse applicator according to claim 9, characterized in that the electrodes are fenestrated.

11. Electric pulse applicator according to claim 10, characterized in that the needle holder (40) carries a syringe (50) associated with each needle, said syringe being formed of a body (51) and a piston (52).

12. Electric pulse applicator according to any one of claims 1 to 11, characterized in that the selector switch (11) comprises two relays (19₁, 20₁, ...) associated with each needle (1), said selector switch being able to connect the needle to the positive terminal or negative terminal of the pulse generator (10).

13. Electric pulse applicator according to any one of claims 8 to 12, characterized in that it comprises a control unit (12) which can produce an electric pulse sequence as determined by the operator, following the injection of a substance.

## Patentansprüche

1. Vorrichtung zur Abgabe elektrischer Impulse für die Behandlung von biologischen Geweben, welche das Aufbringen eines elektrischen Feldes auf Zellen biologischen Gewebes ermöglicht, um die Eigenschaften ihrer Membranen zu verändern, mit Elektroden und einem Impulsgenerator (10),
dadurch gekennzeichnet, daß die Elektroden wenigstens drei Nadeln (1, 2, ..., n), die zum Einführen in das zu behandelnde Gewebe und zum Begrenzen eines Behandlungsvolumens vorgesehen sind, wobei die Nadeln (1, 2, ..., n) jeweils zu zweit Nadelpaare bilden, und einen Nadelschalter (11) aufweisen, der die von dem Impulsgenerator (10) erzeugten Impulse nacheinander an die verschiedenen Nadelpaare leitet.

2. Vorrichtung zur Abgabe elektrischer Impulse nach Anspruch 1, dadurch gekennzeichnet, daß die Nadeln (1, 2, ..., n) der Elektroden austauschbar an einem Nadelapplikator (40) angebracht sind.

3. Vorrichtung zur Abgabe elektrischer Impulse nach Anspruch 2, dadurch gekennzeichnet, daß die Nadeln (1, 2, ..., n) jeweils eine Basis (30) und einen in einer Spitze endenden Schaft (34) aufweisen, wobei die Basis (30) die Befestigung der Nadel an dem Nadelhalter (40) und die Spitze (35) das Eindringen der Nadel in die Gewebe ermöglicht, und wobei ein oder mehrere Bereiche des Schafts von einer isolierenden Hülle (36) umgeben sind.

4. Vorrichtung zur Abgabe elektrischer Impulse nach Anspruch 3, dadurch gekennzeichnet, daß ein Teil der isolierenden Hüllen (36) der Nadeln (1, 2, ..., n) zum implantierten Verbleib in den Geweben bestimmt ist.

5. Vorrichtung zur Abgabe elektrischer Impulse nach einem der Ansprüche 1 bis 4, dadurch gekennzeichnet, daß die Elektroden eine mittige Nadel (1) und sechs Randnadeln (2-n) aufweist, die in einem auf die mittige Nadel (1) zentrierten Kreis gleichmäßig beabstandet angeordnet sind.

6. Vorrichtung zur Abgabe elektrischer Impulse nach einem der Ansprüche 1 bis 5, dadurch gekennzeichnet, daß die Spannung der aufgebrachten Impulse zu der Entfernung zwischen den beiden Nadeln proportional ist, zwischen denen der Impuls erzeugt wird.

7. Vorrichtung zur Abgabe elektrischer Impulse nach einem der Ansprüche 1 bis 6, dadurch gekennzeichnet, daß jede Nadel von den ihr benachbarten Nadeln um eine Entfernung zwischen 4 und 10 mm, und vorzugsweise 6,5 mm, beabstandet ist.

8. Vorrichtung zur Abgabe elektrischer Impulse nach einem der Ansprüche 1 bis 7, dadurch gekennzeichnet, daß jeder elektrische Impuls ein Rechteckimpuls mit einer Amplitude zwischen 100 und 1500 V und einer Zeitdauer zwischen 10 und 200 µs ist.

9. Vorrichtung zur Abgabe elektrischer Impulse nach einem der Ansprüche 1 bis 8, dadurch gekennzeichnet, daß die Nadeln (1, 2, ..., n) der Elektroden hohl sind und das lokale Injizieren der aktiven Substanz in das Behandlungsvolumen ermöglichen.

10. Vorrichtung zur Abgabe elektrischer Impulse nach Anspruch 9, dadurch gekennzeichnet, daß die Elektroden mit Fenstern versehen sind.

11. Vorrichtung zur Abgabe elektrischer Impulse nach Anspruch 10, dadurch gekennzeichnet, daß der Nadelhalter (40) eine aus einem Körper (51) und einem Kolben (52) bestehende Spritze (50) trägt, die mit jeder der Nadeln verbunden ist.

12. Vorrichtung zur Abgabe elektrischer Impulse nach einem der Ansprüche 1 bis 11, dadurch gekennzeichnet, daß der Schalter (11) zwei Relais (19₁, 20₁, ...) aufweist, die mit jeder Nadel (1) verbunden sind, wodurch diese gegebenenfalls mit dem positiven oder dem negativen Anschluß des Impulsgenerators (10) verbunden werden kann.

13. Vorrichtung zur Abgabe elektrischer Impulse nach einem der Ansprüche 8 bis 12, dadurch gekennzeichnet, daß sie eine Steuerung (12) aufweist, die nach dem Injizieren der Substanz das Aufbringen einer beliebigen, von dem Bediener bestimmten Folge elektrischer Impulse ermöglicht.
